# EUROPÄISCHE PATENTSCHRIFT

(11) **EP 0 607 231 B1**
(45) Veröffentlichungstag und Bekanntmachung des Hinweises auf die Patenterteilung: **27.08.1997**
(21) Anmeldenummer: 92921012.8
(22) Anmeldetag: 02.10.1992
(51) Int. Cl.: A61K 31/20

(54) **VERWENDUNG EINER EMULSION, ZUR HERSTELLUNG EINES ARZNEIMITTELS ZUR I.V.-VERABREICHUNG ZUR BEHANDLUNG VON HAUTERKRANKUNGEN**
USE OF AN EMULSION TO PREPARE AN INTRAVENOUSLY ADMINISTERED MEDICAMENT FOR TREATING SKIN DISEASES
UTILISATION D'UNE EMULSION AFIN DE PREPARER UN MEDICAMENT ADMINISTRE PAR VOIE INTRA-VEINEUSE POUR TAITER DES MALADIES DE LA PEAU

(30) Priorität: 11.10.1991 DE 4133694
(43) Veröffentlichungstag der Anmeldung: 27.07.1994
(73) Patentinhaber: Fresenius AG, 61350 Bad Homburg (DE)
(72) Erfinder: SCHLOTZER, Ewald, D-6370 Oberursel (DE); MAYSER, Peter, D-6301 Biebertal 1 (DE); GRIMMINGER, Friedrich, D-6308 Butzbach 12 (DE); SEEGER, Werner, D-35633 Lahnau (DE); WEIDLER, Burghard, D-61239 Ober-Mörlen (DE); SOMMERMEYER, Klaus, D-6365 Rosbach (DE); THOMAS, Martin, D-6000 Frankfurt am Main 70 (DE)
(74) Vertreter: Luderschmidt, Wolfgang, Dr. phil.nat.
(86) Internationale Anmeldenummer: EP9202285
(87) Internationale Veröffentlichungsnummer: WO9306812

(56) Entgegenhaltungen:
- EP-A- 0 298 293
- EP-A- 0 311 091
- WO-A-90/08544
- J. NUTR., Bd. 119, Nr. 4, 1989, Seiten 521-528; A.P. SIMOPOULOS: "Summary of the NATO advanced research workshop oon dietary w3 and w6 fatty acids: biological effects and nutritional essentiality
- ARCH. DERMATOL., Bd. 122, Nr. 11, 1982, Seiten 1277-1282; V.A. ZIBOH ET AL.: 'Effects of dietary supplementation of fish oil on neutrophil and epidermal fatty acids'
- MED. HYPOTHESES, Bd. 22, Nr. 4, 1987, Seiten 421-428; D.F. HORROBIN: 'Low prevalence of coronary heart disease (chd), psoriasis, asthma and rheumatoid arthritis in eskimos: are they caused by high dietary intake of eicosapentaenoic acid (epa), a genetic variation of essential fatty acid (efa) metabolism or a combination of both?'
- J. NUTR., Bd. 117, Nr. 8, 1987, Seiten 1360-1370; R.S.CHAPKIN ET AL.: 'Dietary influences of evening primrose and fish oil on the skin of essential fatty acid-deficient guinea pigs.'
- J. INVEST. DERMATOL., Bd. 70, Nr. 4, 1978, Seiten 200-203; N.J. LOWE ET AL.: 'Linoleic acid effects on epidermal DNA synthesis and cutaneous prostaglandin levels in essential fatty acid deficiency'
- LIPIDS, Bd. 22, Nr. 3, 1987, Seiten 133-138; M. MARTINEZ ET AL.: 'Effects of parenteral nutrition with high doses of linoleate on the developing human liver and brain.'

## Beschreibung

### Technisches Gebiet

Die Erfindung betrifft die Verwendung einer Emulsion, die eine oder mehrere mehrfach ungesättigte langkettige Omega-3-Fettsäuren und/oder Omega-6-Fettsäuren oder deren pharmazeutisch verträgliche Ester oder Salze enthält zur i.v.-Verabreichung zur Behandlung von Hauterkrankungen bzw. zur Herstellung eines Arzneimittels zur i.v.-Verabreichung zur Behandlung von Hauterkrankungen, besonders entzündlichen Hauterkrankungen sowie Erkrankungen der Dermatitis- und Ekzemgruppe, insbesondere Erkrankungen der Dermatitis- und Ekzemgruppe.

Hautkrankheiten stellen heute einen hohen Prozentsatz der Erkrankungen bei Mensch und Tier dar, wobei z.B. Psoriasis zu den häufigsten Hauterkrankungen zählt, an der etwa 1 bis 2 % der Bevölkerung erkranken.

### Stand der Technik

Die Notwendigkeit essentieller Fettsäuren für Aufbau und Funktion der Haut ist bekannt. Im Tiermodell zeigten Ratten, die eine von diesen Fettsauren freie Diät erhielten, Entwicklungsstörungen und Hautveränderungen mit Rötung, Schuppung und Hyperkeratosen im Bereich der Talgdrüsen. Weitere Veränderungen waren vermehrtes Effluvium, Hyperproliferation mit gesteigertem epidermalem Zellturnover, gestörte Wundheilung und vermehrter transepidermaler Wasserverlust. Nach Substitution waren diese Hautveränderungen reversibel. Hauptlieferanten der essentiellen Fettsäuren, die nach der Position ihrer ersten Doppelbindung in Omega-3- und Omega-6-Fettsäuren unterschieden werden, sind vornehmlich Kaltwasserfische (für Omega-3-Fettsäuren) bzw. pflanzliche Öle (für Omega-6-Fettsäuren). Im Säugerorganismus können durch differente Desaturasen und Elongasen weitere Folgeprodukte gebildet werden.

Die Beobachtung, daß Bevölkerungsgruppen, die einen hohen Konsum an Omega-3-Fettsäuren haben (z.B. die Eskimos), nur ein zwanzigstel der Psoriasisinzidenz vergleichbarer Bevölkerungsgruppen haben, welche sich vornehmlich von Omega-6-Fettsäuren ernähren, führten zu mehreren klinischen Studien, die die Wirkung einer fischölreichen Diät, d.h. einer oralen Fischölapplikation auf den Verlauf verschiedener Formen von Psoriasis untersuchten (z.B. The Lancet, February 20, 1988, Seite 378, J.Am.Acad.Dermatol. 1988, Band 18, Seiten 1267 bis 1273, Brit. J. Dermatol. 1987, 117, Seiten 599-613).

Bei Psoriasis vulgaris vom chronisch-stationären Typ sind die Aussagen uneinheitlich, stimmen jedoch darin überein, daß die klinische Besserung einer linearen Dosis-Wirkungsbeziehung folgt. Bei den exsudativen Formen (Psoriasis exanthematica, Psoriasis pustulosa), aber auch bei Psoriasis arthropathica scheinen sich im Trend einheitlich positive Aussagen abzuzeichnen, jedoch sind die publizierten Fallzahlen noch sehr gering. Zugrunde liegt diesem therapeutischen Ansatz die Suppression und Antagonisierung des in der Pathogenese bedeutsamen Arachidonsäurestoffwechsels durch Einschleusung der strukturverwandten Eicosapentaensäure (EPA) in den Lipidstoffwechsel von sowohl Keratinozyten als auch neutrophilen Granulozyten. Granulozyten scheinen insbesondere bei entzündlichen Psoriasis-Formen eine bedeutsame Rolle zu spielen, was durch gesteigerte Funktionsparameter sowie die histologischen Charakteristika der Infiltration der Epidermis und Bildung von sogenannten Munroeschen Mikroabzessen unterstützt wird. Die gesteigerte chemotaktische und proinflammatorische Aktivität resultiert insbesondere bei pustulösen Formen in der Bildung klinisch sichtbarer Pusteln auf entzündlich veränderter Haut.

LTB4 als ein Lipoxygenaseprodukt der Arachidonsäure, welches in psoriatischen Läsionen vermehrt gefunden wurde, kann als potente chemotaktische Substanz diese Befunde erklären. Es stimuliert zudem die Keratinozytenproliferation in der Zellkultur.

Durch orale Aufnahme von Eicosapentaensäure (Omega-3-Fettsäure), die in Fischöl enthalten ist, kann der Metabolismus der Arachidonsäure (Omega-6-Fettsäure) kompetitiv dergestalt gehemmt werden, daß biologisch weniger potente Metaboliten entstehen, im Falle des LTB4 beispielsweise das chemotaktisch wesentlich schwächer wirksame LTB5. Erklärt wird dies durch die Aufnahme von Eicosapentaensäure anstelle von Arachidonsäure in die Zellmembran und durch die Kompetition dieser Substanz um die Enzyme Cyclooxygenase und Lipoxygenase. Die durchgeführten Untersuchungen zeigten, daß die orale Behandlung von Hauterkrankungen mit Fischöl (z.B. in Form der oralen Verabreichung von Fischölkapseln) eine lange Behandlungsdauer (bis zu mehreren Monaten) erfordern, in deren Verlauf täglich z. T. sehr hohe Fischölmengen (z.B. 10 bis 75 g) aufgenommen werden müssen, wobei im Verlauf der Behandlung entsprechend erhebliche gastrointestinale Probleme (z.B. Übelkeit, Völlegefühl, Brechreiz, Aufstoßen) auftreten. Ein weiterer Aspekt einer solchen oralen Fischöl-Therapie ist die schlechte Patienten-Compliance.

Andere Behandlungsformen der Hauterkrankungen, einschließlich schwerer Psoriasis sind die Behandlung mit Retinoiden, z.B. Eretinat und Acitretin. Wesentliche Nachteile dieser Behandlung sind Hyperlipidämie, einschließlich Hypertriglyceridämie, Hypercholesterinämie und verringerte Gehalte an High-Density-Lipoprotein-Cholesterin (HDL-C). Die durch Retinoidbehandlung induzierten Nebenwirkungen stellen bei längerer oraler Therapie ein potentes Risiko für schwere cardio-vaskuläre Erkrankungen dar. Andere bisher bekannte Behandlungsweisen von Hauterkrankungen sind die Behandlung mit Cignolin, nicht-steroidalen Antiphlogistika, Antihistaminika oder Corticosteroiden sowie die Photo- bzw. Balneophototherapie. Doch auch diese Behandlungsweisen führen zu erheblichen Nebenwirkungen, wobei als häufigste Nebenwirkung bei der Therapie mit Cignolin und der Photo- bzw. Balneophototherapie Verbrennungen auftreten und bei Behandlung mit Corticosteroiden Hautathrophie beobachtet wird. Allen diesen bekannten Standardtherapien, mit Ausnahme der Therapie mit Corticosteroiden, gemeinsam ist jedoch der Nachteil, daß sie eine recht lange Behandlungsdauer erfordern. Corticosteroide wirken sehr rasch, sind jedoch, da sie zu Hautatrophie führen, nur kurzzeitig einsetzbar. Behandlungen mit nicht-steroidalen Antiphlogistika oder mit Antihistaminika stellen zudem lediglich lindernde Maßnahmen dar, die als Nebenwirkungen zu Magenbeschwerden führen bzw. Müdigkeit bewirken.

### Darstellung der Erfindung

Aufgabe der Erfindung ist daher, ein geeignetes Mittel und Verfahren zur Behandlung von Hauterkrankungen, besonders entzündlichen Hauterkrankungen sowie Erkrankungen der Dermatitis- und Ekzemgruppe, insbesondere Erkrankungen der Dermatitis- und Ekzemgruppe, das die Nachteile der bekannten Mittel und Verfahren nicht aufweist und mit dessen Hilfe innerhalb von kürzerer Behandlungsdauer sichtbare Heilerfolge erzielt werden können.

Erfindungsgemäß wurde überraschend festgestellt, daß durch die i.v.-Verabreichung von Fettemulsionen, die ein oder mehrere mehrfach ungesättigte, langkettige Omega-3-Fettsäuren und/oder Omega-6-Fettsäuren oder deren pharmazeutisch verträgliche Ester oder Salze enthalten, zur Behandlung von Hauterkrankungen, besonders entzündlichen Hauterkrankungen sowie Erkrankungen der Dermatitis- und Ekzemgruppe, insbesondere Erkrankungen der Dermatitis- und Ekzemgruppe, bereits nach wenigen Behandlungstagen ein Therapieerfolg sichtbar ist und die mit den bekannten Standardtherapien verbundenen Nachteile bzw. Nebenwirkungen vermieden werden können.

Neben dem schnellen Wirkungseintritt bei i.v.-Behandlung bestehen gegenüber den bisherigen Standardbehandlungsweisen weitere Vorteile in der besseren Patienten-Compliance, d.h. der besseren Akzeptanz und Bereitschaft der Patienten zur Therapie, einer geringeren Belastung der Patienten durch die i.v.-Verabreichung, keinem Auftreten gastrointestinaler Probleme sowie einem kürzeren Krankenhausaufenthalt stationärer Patienten, einer kürzeren Behandlungsdauer und damit in einer Senkung der Behandlungskosten. Während die vorstehend beschriebene orale Behandlung der Hauterkrankungen mehrere Wochen (mindestens 6 Wochen) bis mehrere Monate bei Verabreichung von täglich 20 und mehr Fischölkapseln erfordert, genügen bei i.v.-Behandlung mit der erfindungsgemäß angewandten Emulsion wenige Behandlungstage bei einer Infusionsdauer von täglich etwa einer Stunde.

Bevorzugt werden in den erfindungsgemäß verwendeten Emulsionen solche mehrfach ungesättigten, langkettigen Omega-3- und/oder Omega-6-Fettsäuren, die 18 bis 22 C-Atome enthalten, sowie deren Ester und Salze. Beispiele für geeignete Omega-3-Fettsäuren sind α-Linolensäure, Eicosapentaensäure (EPA) und Docosahexaensäure (DCHA), wobei bevorzugt EPA und DCHA und insbesondere EPA verwendet wird. In den Emulsionen können eine oder mehrere der Omega-3-Fettsäuren vorhanden sein. Die Säuren oder deren pharmazeutisch verträglichen Ester oder Salze können entweder in Reinform oder als Bestandteil von Fischöl, hochgereinigten Fischölkonzentraten oder Leinöl, vorzugsweise von Fischöl oder hochgereinigten Fischölkonzentraten eingesetzt werden. Geeignete Fischöle sind beispielsweise solche, wie sie technisch in bedeutendem Umfang aus Kaltwasserfischen gewonnen werden. Beispiele für solche Fischöle sind Pilchardöl, Menhadenöl, Perufischöl, Sardinenöl, Lachsöl, Heringsöl und Makrelenöl. Bevorzugt sind hochgereinigte Fischölkonzentrate, die beispielsweise aus Makrele, Sardine, Hering oder Lachs gewonnen werden, wobei diese einen EPA-Gehalt von 20 bis 40 %, vorzugsweise mindestens 26 % (bezogen auf die Fettsäuremethylester des Fischölkonzentrats) besitzen. Beispiele für geeignete Fischölemulsionen sind in der DE-PS 37 22 540, auf die verwiesen wird, beschrieben.

Beispiele für geeignete Omega-6-Fettsäuren sind Linolsäure, γ-Linolensäure` Dihomo-γ-linolensäure und Arachidonsäure, wobei γ-Linolensäure und Dihomo-γ-linolensäure bevorzugt sind. In der erfindungsgemäß angewandten Emulsion können eine oder mehrere Omega-6-Fettsauren enthalten sein. Omega-6-Fettsäuren oder deren pharmazeutisch verträglichen Ester oder Salze können entweder in Reinform oder in Form von Bestandteilen von Ölen, beispielsweise Nachtkerzenöl, Borretschöl oder Sojaöl verwendet werden. Vorzugsweise wird Nachtkerzenöl verwendet.

Die pharmazeutisch verträglichen Ester und Salze der genannten Omega-3- und/oder Omega-6-Fettsäuren werden bevorzugt verwendet, wobei die pharmazeutisch verträglichen Ester dieser Säuren besonders bevorzugt sind. Pharmazeutisch verträgliche Ester der Omega-3- und Omega-6-Fettsäuren sind der Ethylester oder Glycerinester, z.B. Mono-, Di- oder Tri-glycerinester, wobei Triglyceride bevorzugt sind. Als pharmazeutisch verträgliche Salze geeignet sind die Natriumsalze.

In den erfindungsgemäß verwendeten Emulsionen können entweder
a) Omega-3-Fettsäuren, deren pharmazeutisch verträgliche Ester oder Salze in Reinform oder als Bestandteil von Ölen, wie sie vorstehend genannt wurden, oder
b) Omega-6-Fettsäuren, deren pharmazeutisch verträgliche Ester oder Salze in Reinform oder als Bestandteil von Ölen, wie sie vorstehend ganannt wurden, oder
c) ein Gemisch der vorstehend unter a) und unter b) genannten Säuren oder deren pharmazeutisch verträglichen Estern oder Salzen enthalten sein.

Beispielsweise kann in den erfindungsgemäß verwendeten Emulsionen ein Gemisch aus Fischöl und anderen Ölen, wie Nachtkerzenöl, Borretschöl oder Sojaöl enthalten sein, wobei das Verhältnis von Fischöl zu den anderen Ölen (auf Gewichtsbasis) geeigneterweise im Bereich zwischen 9:1 und 1:9 liegt. Beispielsweise kann das Verhältnis von Fischöl zu Nachtkerzenöl und/oder Borretschöl 1:1 und das Verhältnis von Fischöl zu Sojaöl 7:1 betragen.

Die Omega-3-Fettsäuren und/oder Omega-6-Fettsäuren oder deren pharmazeutisch verträglichen Ester oder Salze sind in den erfindungsgemäß verwendeten Emulsionen in Mengen von 5 bis 45 Gew.-%, vorzugsweise in Mengen von 10 bis 30 Gew.-% und insbesondere in Mengen von 10-20 Gew.-% enthalten.

Bevorzugt sind erfindungsgemäß solche Emulsionen auf Basis von lediglich Omega-3-Fettsäuren, deren Ester oder Salze in Reinform oder in Form von Bestandteilen von Ölen, wie sie vorstehend genannt wurden, insbesondere solche auf Basis von Fischölen.

Die erfindungsgemäß verwendeten Emulsionen enthalten ebenfalls mindestens einen physiologisch unbedenklichen Emulgator. Geeignet sind Phospholipide tierischen oder pflanzlichen Ursprungs, vorzugsweise solche Phospholipide, die als ungesättigte Fettsäure EPA enthalten. Insbesondere geeignet ist Eilecithin.

Der Emulgator liegt in der Emulsion in einer Menge von 5 bis 15 Gew.-% (bezogen auf den Fettgehalt), vorzugsweise in einer Menge von 5 bis 12 Gew.-% (bezogen auf den Fettgehalt) vor.

Ferner können die Emulsionen als Antioxidans Vitamin E, z.B. in Form von Tocopherol oder pharmazeutisch unbedenklichem Tocopherolester, z.B. Tocopherolacetat, in einer Menge von 0,15 bis 1,5 Gew.-% (bezogen auf den Fettgehalt) enthalten.

Als weitere Zusätze können die erfindungsgemäß verwendeten Emulsionen übliche Hilfs- und Zusatzstoffe, wie übliche Emulsionsstabilisatoren, Isotonisierungszusätze und/oder Coemulgatoren sowie gegebenenfalls eine Selenverbindung aufweisen. Als Selenverbindung geeignet ist beispielsweise Na₂SeO₃ x .5H₂O.

Als Isotonisierungszusätze geeignet sind die üblicherweise verwendeten Isotonisierungsmittel, wie z.B. Glycerin, Glucose, Xylit und Sorbit, wobei Glycerin bevorzugt ist.

Eine geeignete bevorzugte erfindungsgemäß verwendete Emulsion besitzt beispielsweise folgende Zusammensetzung:

| | |
|---|---|
| Fischöl | 100 mg/ml |
| Glycerin (Isotonisierungsmittel) | 25 mg/ml |
| Eilecithin | 12 mg/ml |
| Vitamin E | 0,15 mg/ml |
| Wasser (zur Injektion )zum Auffüllen auf 1 ml. | |

Das in der vorstehend genannten Zusammensetzung eingesetzte Fischöl ist vorzugsweise hochraffiniertes Fischöl, das mittels eines spezifischen Verfahrens, wie es in der DE-PS 37 22 540 beschrieben ist, an Omega-3-Fettsäuren im Triglyceridverband angereichert ist. Es enthält mindestens 40 Gew.-% Omega-3-Fettsäuren. Der Gesamtgehalt des Fischöles an EPA und DCHA im Triglyceridverband liegt im Bereich von 25 bis 50 Gew.-%, vorzugsweise im Bereich von 35 bis 50 Gew.-% (jeweils bestimmt als Flächenprozente im Gaschromatogramm). Die EPA und die DCHA können in dem Fischöl in verschiedenen Mengenverhältnissen zueinander vorliegen, die durch Ausmessung der zugehörigen Flächen im Gaschromatogramm bestimmt werden können. Diese Mengenverhältnisse sind abhängig von der Natur des verwendeten Fischöls und dem Grad der erzielten Anreicherung an Omega-3-Fettsauren. Fischöle, bei denen EPA und DCHA im Triglyceridverband im Mengenverhältnis von EPA zu DCHA im Bereich von 0,5 bis 2,6 (Flächenverhältnis im Gaschromatogramm) vorliegen, liefern bevorzugt verwendete Fettemulsionen.

Die erfindungsgemäß verwendeten Fettemulsionen sind Öl-in-Wasser (O/W)-Emulsionen, bei denen die äußere, zusammenhängende Phase aus destilliertem, für i.v.-Verabreichung geeignetem Wasser besteht.

Die Herstellung der erfindungsgemäß verwendeten Emulsionen erfolgt in an sich bekannter Weise. Eine geeignete Verfahrensweise wird beispielsweise in der DE-PS-37 22 540 beschrieben.

Die Emulsionen können erfindungsgemäß zur i.v.-Verabreichung zur Behandlung von Hauterkrankungen, wie
1. Hauterkrankungen, die durch Abkömmlinge der Arachidonsäure induziert oder unterhalten werden, die von Granulozyten bzw. deren Subpopulationen (Neutrophile, Eosinophile), von Keratinozyten oder von beiden gebildet werden, wobei insbesondere zu nennen sind:
   a) entzündliche Hauterkrankungen, wie Psoriasis vulgaris, Psoriasis pustulosa, Psoriasis athropathica, Vasculitis allergica,
   b) Erkrankungen der Dermatitis- und Ekzemgruppe, wie Neurodermitis constitutionalis, Kontaktdermatitiden (allergisch/toxisch),
   c) entzündliche Hauterkrankungen mit Eosinophilie (Hypereosinophilie-Syndrom, Eosinophile Zellulitis, Hypereosinophile Dermatitis),
   d) bullöse Dermatosen, wie Pemphigus vulgaris, bullöses Pemphigoid,
   e) Lichtdermatosen, z.B. akute Lichtdermatitis, polymorphe Lichtdermatose.
2. Hauterkrankungen bei oder mit gestörter Funktion des Immunsystems, insbesondere bei überschießender Immunfunktion, wobei insbesondere zu nennen sind: Lupus erythematodes und andere sogenannte Kollagenosen, Alopezia areata, graft versus host disease, Lichen ruber.

Die Emulsionen werden erfindungsgemäß i.v. besonders bei der Behandlung von entzündlichen Hauterkrankungen sowie Erkrankungen der Dermatitis- und Ekzemgruppe, insbesondere zur Behandlung von Erkrankungen der Dermatitis- und Ekzemgruppe verwendet. Erfindungsgemäß wurde überraschend festgestellt, daß bei i.v.-Applikation von Fettemulsionen neben dem Erreichen höherer Wirkspiegel auch akut-antiinflammatorische Effekte erzielt werden können. Gemäß der bestehenden Auffassung beruht die Wirkung der Omega-3-Fettsäuren, insbesondere die Wirkung der EPA, auf einem Einbau dieser Fettsäuren anstelle von Arachidonsäure in die Zellmembran, aus der sie bei entsprechenden Stimuli durch Phospholipasen freigesetzt und je nach enzymatischer Ausstattung der betreffenden Zelle mit Cyclo- und/oder Lipoxygenasen zu entsprechenden Mediatoren umgesetzt werden. Mit diesem Ansatz sind Effekte nur nach mehrwöchiger Therapiedauer zu erhalten, da erst mit dem komplexen Umweg über eine Modulation der zellulären Phospholipidkomposition Eicosapentaensäure verfügbar wird. Erfindungsgemäß wurde in Bezug auf den therapeutischen Einsatz bei das Hautorgan betreffenden Erkrankungen überraschender Weise gefunden, daß bei intravenöser Applikation einer Fischölemulsion eine akut therapeutische Intervention bei den vorstehend aufgeführten Erkrankungen möglich ist. Dies beruht möglicherweise darauf, daß in einem inflammatorischen Focus freie EPA auch direkt von zur Eicosanoidsynthese befähigten Zellen in Konkurrenz zu freier extrazellulärer Arachidonsäure aufgenommen und verstoffwechselt werden kann. Daher ist die akut antiinflammatorische therapeutische Intervention eine neue Möglichkeit in der Behandlung der vorstehend aufgeführten Erkrankungen.

In Bezug auf die aufgeführten Erkrankungen ergeben sich akut-immunmodulatorische Effekte über die Veränderung des Mediatorenprofils (Cyclo- und Lipoxygenaseprodukte) sowie der Zusammensetzung der Zellmembran, da sich Fluidität und damit die Moglichkeit der Antigenpräsentation der Zellmembran mit der Zusammensetzung ihres Lipidanteils ändert. Ferner ändern sich unter der Substitution von Omega-3-Fettsäuren Eigenschaften von Lymphozyten (z.B. Suppression von Killer-Zellen) und Makrophagen (reduzierte Eicosanoidproduktion bei unveränderter Phagozytosefähigkeit und Produktion von Sauerstoffradikalen).

Die erfindungsgemäße i.v.-Verabreichung der Emulsionen bei Hauterkrankungen kann ferner im Rahmen einer Kombinationstherapie erfolgen, insbesondere in Kombination mit einer Therapie mit
a) Retinoiden systemisch,
b) Cignolin extern,
c) Phototherapie (SUP, PUVA) bzw. Balneophototherapie,
d) Corticosteroiden (intern/extern),
e) nicht-steroidaler Antiphlogistika und/oder
f) Antihistaminika.

So wird, wenn bei Psoriasis und anderen entzündlichen Hauterkrankungen Retinoide verwendet werden, durch die zusätzliche i.v.-Applikation von Omega-3-Fettsäuren neben dem zusätzlichen antiinflammatorischen Effekt eine rasche Absenkung der durch Retinoid induzierten Serumlipiderhöhung beobachtet. In der Kombinationstherapie von i.v.-Verabreichung der Emulsionen und Photo- bzw. Cignolintherapie kann die Erythemschwelle erhöht werden, so daß eine schnellere Dosissteigerung der herkömmlichen Therapeutika möglich wird bzw. deren unerwünschte Nebenwirkungen gemildert werden. Durch die Kombination von i.v.-Verabreichung der Emulsionen mit der üblichen Behandlung mit Corticosteroiden wird die sonst erforderliche Dosis von Corticosteroiden herabgesetzt und folglich die durch diese bedingte Hautatrophie gemildert. Die Kombination der i.v.-Verabreichung der Emulsionen mit nicht-steroidalen Antiphlogistika oder Antihistaminika ermöglicht entsprechend die Herabsetzung der üblichen, sonst erforderlichen Dosis und Zeitdauer der Verabreichung der Mittel und damit auch die Reduktion der sonst mit ihrer Verwendung verbundenen Nachteile.

Für die i.v.-Verabreichung zur Behandlung von Hauterkrankungen geeignet ist eine solche Menge an Fettemulsion, die 0,01 bis 0,3 g, vorzugsweise 0,05 bis 0,15 g der genannten Fettsäure(n), deren Estern oder Salzen /kg Körpergewicht und Tag entsprechen. So kann beispielsweise eine solche Menge an Fettemulsion verwendet werden, die 0,01 bis 0,2 g, vorzugsweise 0,05 bis 0,1 g EPA, deren Estern oder Salzen /kg Körpergewicht und Tag entspricht, oder die 0,05 bis 0,5 g Öl (z.B. Fischöl und/oder Nachtkerzenöl) /kg Körpergewicht und Tag, vorzugsweise 0,1 bis 0,5 g, insbesondere 0,1 bis 0,3 g Fischöl /kg Körpergewicht und Tag entspricht.

Die erfindungsgemäß verwendeten Fettemulsionen sind nicht toxisch.

### Wege zur Ausführung der Erfindung

Die nachfolgenden Beispiele dienen der weiteren Erläuterung der vorliegenden Erfindung.

### Beispiel 1

Aus den nachfolgend ausgeführten Bestandteilen wurde eine für die i.v.-Verabreichung geeignete Fettemulsion hergestellt:

| | |
|---|---|
| Fischöl | 100 mg |
| Glycerin | 25 mg |
| Eilecithin | 12 mg |
| Vitamin E | 0,15 mg |
| Wasser (zur Injektion) aufgefüllt auf 1 ml. | |

Das verwendete Fischöl ist hochraffiniert und enthält mindestens 40 Gew.-% Omega-3-Fettsäuren und wurde wie in Beispiel 1 der DE-PS 37 22 540 beschrieben, hergestellt.

Die Fettemulsion wurde in der gleichen Weise hergestellt, wie sie in Beispiel 5 der DE-PS 37 22 540 beschrieben wurde.

Die toxikologische Prüfung der vorstehend hergestellten 10 %-igen Fischölemulsion, untersucht an zwei Spezies, Beagle-Hunden und Charles-River-Ratten, während einer Dauer von 4 Wochen ergab, daß diese Fischölemulsion bei intravenöser Verabreichung bei Dosen bis zu 5000 mg/kg Körpergewicht und Tag keinerlei Hinweise auf eine systemische Toxizität oder intravasculäre Reizung zeigte.

### Beispiel 2

Aus den nachfolgend aufgeführten Bestandteilen wurde eine für die i.v.-Verabreichung geeignete 10-%ige Fettemulsion hergestellt:

| | |
|---|---|
| Sojaöl | 100 mg/ml |
| Glycerin | 25 mg/ml |
| Eilecithin | 12 mg/ml |
| Wasser (zur Injektion) zum Auffüllen auf | 1 ml |

Die in Beispiel 1 und 2 hergestellten Fettemulsionen wurden in der folgenden Weise hinsichtlich ihrer Wirksamkeit bei i.v.-Verabreichung bei Hauterkrankungen untersucht:

Zehn Patienten mit akut exanthematischer Psoriasis wurden in die Untersuchungen einbezogen, wobei bei sechs der Patienten die klinische Erprobung der Emulsion gemäß Beispiel 1 erfolgt, während vier Patienten die Emulsion gemäß Beispiel 2 erhielten. Die einzelnen Patientendaten sind in der Tabelle 1 zusammengestellt.

Die Untersuchung erfolgte unter Doppelblind-Bedingungen. Den Patienten wurde über 10 Tage zweimal täglich im Abstand von 12 Stunden 50 ml der jeweiligen Fettemulsion (Emulsion von Beispiel 1 oder 2) intravenös verabreicht. Die Patienten wurden täglich hinsichtlich der Änderung des Krankheitsbildes und an bestimmten Meßpunkten hinsichtlich der Änderung der EPA-Metaboliten, des Triglyceridgehaltes, des Cholesteringehaltes und des IgE nach bekannten Methoden untersucht. Die Bestimmung der EPA-Metaboliten erfolgte mit Hilfe der High Performance Liquid Chromatography (HPLC), und zwar sowohl nach der reversed als auch nach dem straight phase Verfahren, die Triglyceride und das Cholesterin wurden mittels enzymatischer Farbtests (GPO-PAP-Methode bzw. CHOD-PAP-Methode) bestimmt. Die Beurteilung des Krankheitsbildes erfolgte nach den folgenden Kriterien:
Erythem, Schuppung, Exsudation, subjektive Besserung und Besserung des Juckreizes.

Die einzelnen, bei der Untersuchung erhaltenen Ergebnisse, ausgedrückt als Score-Mittelwerte, sind folgende:

### Score-Mittelwerte (A1-A10)

### 1. Erythem

| Tag | 1 | 2 | 3 | 4 | 5 |
|---|---|---|---|---|---|
| T (6) | 32,6 | 31,8 | 26 | 25,8 | 23,5 |
| K (4) | 29 | 28,5 | 26,75 | 26,5 | 24,5 |

| Tag | 6 | 7 | 8 | 9 | 10 |
|---|---|---|---|---|---|
| T (6) | 22,5 | 21,3 | 19,8 | 17,3 | 16,8 |
| K (4) | 24 | 22,75 | 22 | 21,75 | 20,5 |

### Relativer Score (%)

| Tag | 1 | Tag 10 |
|---|---|---|
| T (6) | 100 | 51,5 |
| K (4) | 100 | 70,7 |

### 2. Schuppung

| Tag | 1 | 2 | 3 | 4 | 5 |
|---|---|---|---|---|---|
| T (6) | 29 | 27,3 | 24,5 | 23,5 | 20,3 |
| K (4) | 20,5 | 18,25 | 16,75 | 16,75 | 16,25 |

| Tag | 6 | 7 | 8 | 9 | 10 |
|---|---|---|---|---|---|
| T (6) | 20,1 | 18 | 16,8 | 15,5 | 15,2 |
| K (4) | 16,25 | 15,5 | 15,25 | 15,25 | 15,25 |

### Relativer Score (%):

| Tag | 1 | Tag 10 |
|---|---|---|
| T (6) | 100 | 52,4 |
| K (4) | 100 | 74,4 |

### 3. Exsudation

| Tag | 1 | 2 | 3 | 4 | 5 |
|---|---|---|---|---|---|
| T (5) | 32,4 | 31,4 | 28,4 | 27,8 | 26 |
| K (2) | 9,25 | 9,75 | 9,75 | 9,75 | 9,75 |

| Tag | 6 | 7 | 8 | 9 | 10 |
|---|---|---|---|---|---|
| T (5) | 25,2 | 22,4 | 21,4 | 18,8 | 17,6 |
| K (2) | 9,75 | 9,75 | 9,75 | 9,75 | 9,75 |

### Relativer Score (%):

| Tag | 1 | Tag 10 |
|---|---|---|
| T (5) | 100 | 54,3 |
| K (2) | 100 | 105 |

Anm: Bei einem der Patienten, die mit Emulsion von Beispiel 1 behandelt wurden, sowie bei zweien der Gruppe, die mit Emulsion von Beispiel 2 behandelt wurde, konnte eine exsudative Komponente nicht nachgewiesen werden.
T = Emulsion von Beispiel 1
K = Emulsion von Beispiel 2.

### 4. Subjektive Besserung

| Tag | 1 | 2 | 3 | 4 | 5 |
|---|---|---|---|---|---|
| T (6) | 18,8 | 18 | 22 | 23,5 | 23,3 |
| K (4) | 23,25 | 24,25 | 23 | 23,75 | 23,25 |

| Tag | 6 | 7 | 8 | 9 | 10 |
|---|---|---|---|---|---|
| T (6) | 24,1 | 24,8 | 27,6 | 28,5 | 28,8 |
| K (4) | 23,5 | 24 | 22,25 | 23,75 | 23,75 |

### Relativer Score (%):

| Tag | 1 | Tag 10 |
|---|---|---|
| T (6) | 100 | 153 |
| K (4) | 100 | 102 |

### 5. Besserung des Juckreizes

| Tag | 1 | 2 | 3 | 4 | 5 |
|---|---|---|---|---|---|
| T (6) | 3,5 | 3 | 4,5 | 5,5 | 5,1 |
| K (4) | 3,5 | 3,5 | 3,25 | 3,75 | 3,75 |

| Tag | 6 | 7 | 8 | 9 | 10 |
|---|---|---|---|---|---|
| T (6) | 5,3 | 5,1 | 5,2 | 5 | 5,8 |
| K (4) | 3,75 | 3,75 | 4 | 3,75 | 4 |

### Relativer Score (%):

| Tag | 1 | Tag 10 |
|---|---|---|
| T (6) | 100 | 165,7 |
| K (4) | 100 | 114 |

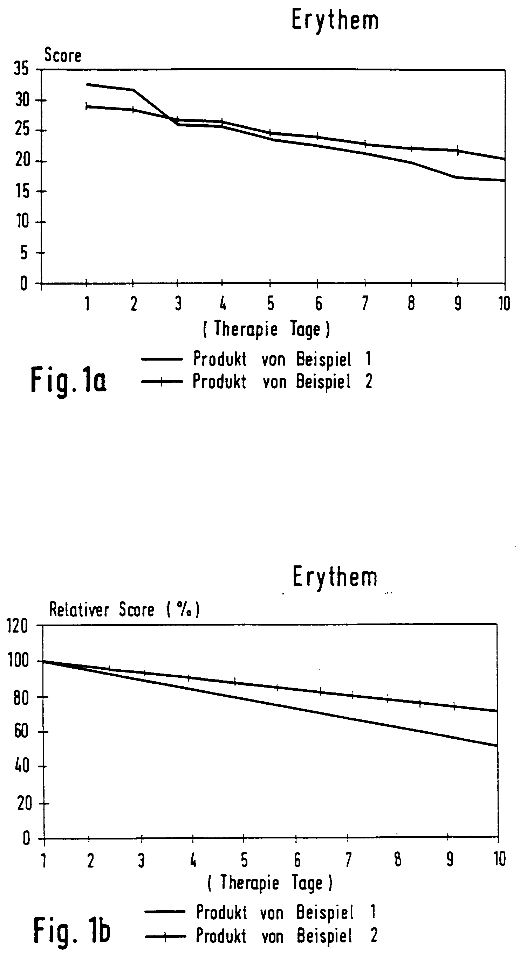

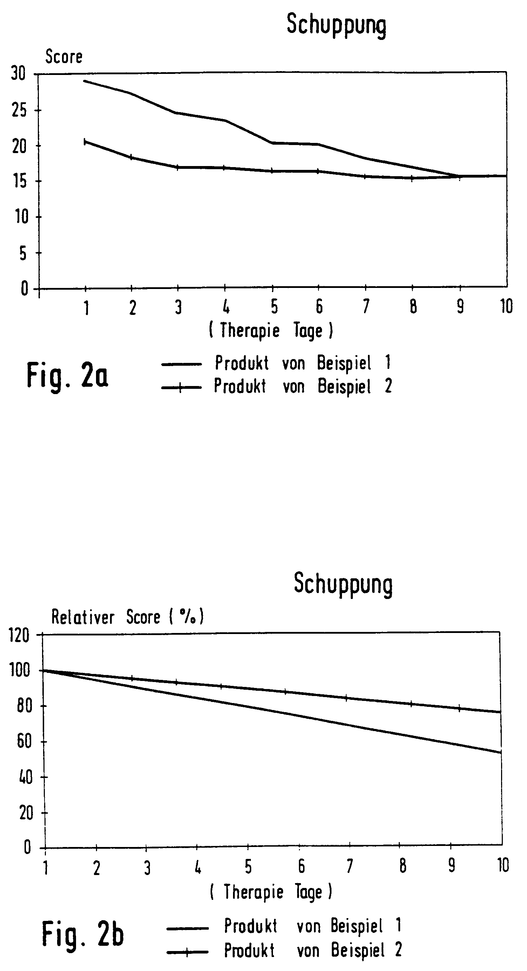

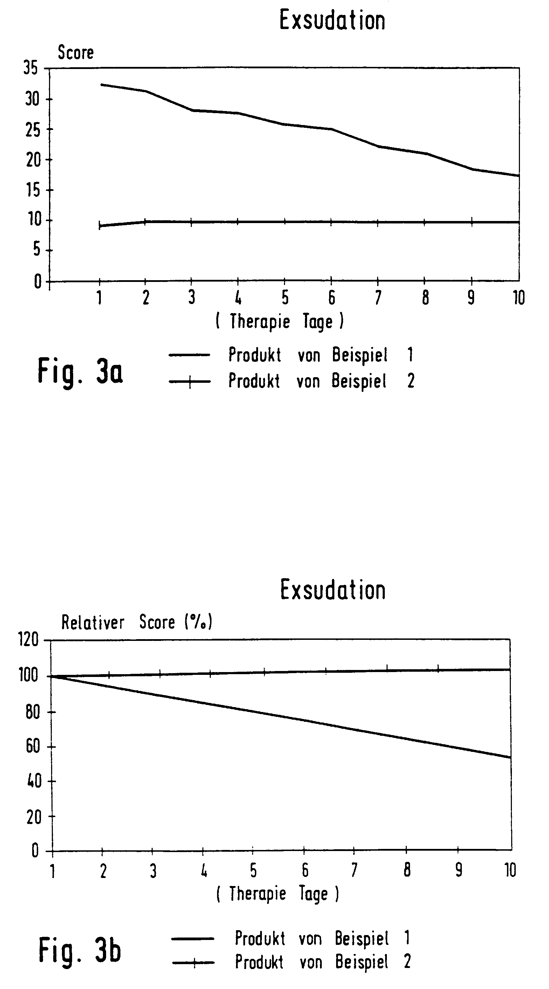

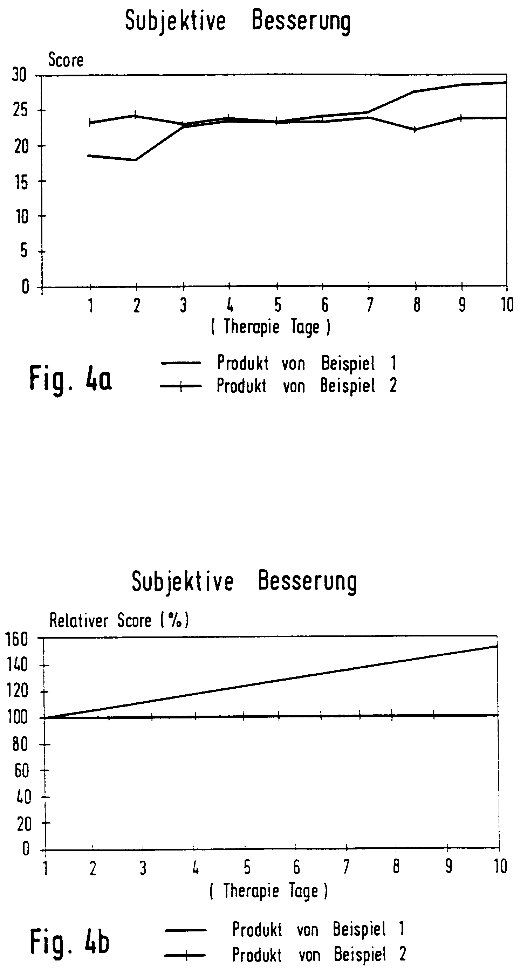

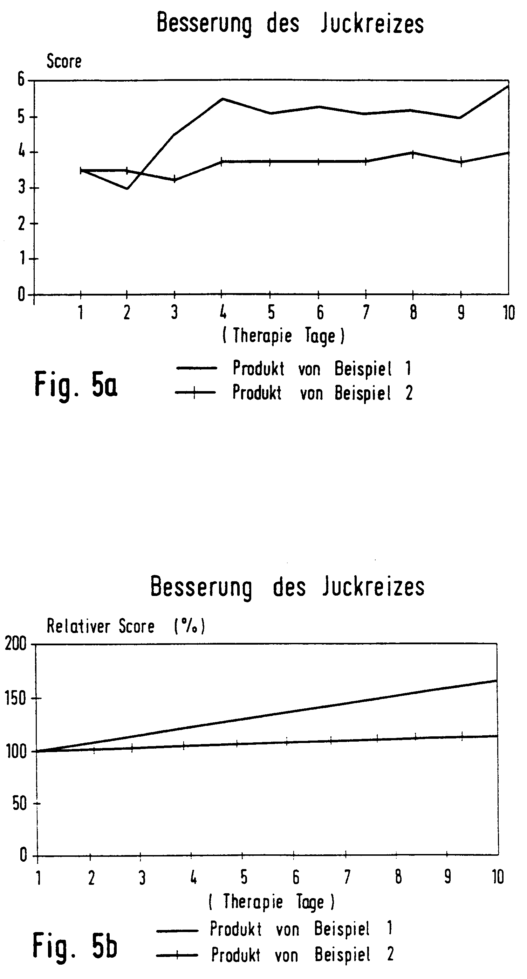

Anm.:
1. Bei den Merkmalen 1-4 ist eine maximale Scorezahl von 50 zu erreichen, bei Merkmal 5 eine maximale Zahl von 10.
2. Das Merkmal 5 (Besserung des Juckreizes) ist Bestandteil des Merkmales 4 (subjektive Besserung), d.h. es geht auch in diese Wertung ein.
3. Bei den Merkmalen 1-3 bedeutet eine abnehmende Scorezahl eine Besserung, bei den Merkmalen 4 und 5 eine Verschlechterung.Zur Verdeutlichung sind die Scoremittelwerte des jeweiligen Erscheinungsbildes der Haut bzw. des Befindens in den Figuren 1 bis 5 wiedergegeben. In der Tabelle 2 sind außerdem die Scoreänderungen sowie die bei den Untersuchungen erhaltenen Werte für EPA-Metaboliten, Triglyceridgehalt, Cholesterin und IgE zusammengestellt.

Die bei den Untersuchungen erhaltenen Ergebnisse zeigen, daß bei der i.v.-Verabreichung der Emulsion gemäß Beispiel 1 bei allen Patienten bereits nach wenigen Tagen eine deutliche Besserung des Krankheitszustandes und bei der i.v.-Verabreichung der Emulsion gemäß Beispiel 2 bei allen Patienten bereits nach wenigen Tagen eine mäßige Besserung des Krankheitszustandes auftrat. Die Besserung des Krankheitszustandes korrelierte mit dem Anstieg der EPA-Metaboliten und zu dem mit der absoluten Höhe des Serum-IgE-Wertes, so daß insbesondere eine Neurodermitis mit der erfindungsgemäßen i.v.-Behandlung beeinflußt werden kann. Außer einer leichten Venenreizung wurden keine Nebenwirkungen beobachtet.

### Beispiel 3

Das Beispiel 1 wurde wiederholt mit der Ausnahme, daß anstelle von Fischöl die gleiche Menge Nachtkerzenöl eingesetzt wurde.

### Beispiel 4

Das Beispiel 1 wurde wiederholt mit der Ausnahme, daß 50 mg des in Beispiel 1 eingesetzten Fischöls durch 50 mg Nachtkerzenöl ersetzt wurden. Bei der i.v.-Verabreichung der gemäß diesem Beispiel erhaltenen Fettemulsion wurden ähnliche Untersuchungsergebnisse erhalten, wie sie vorstehend für Beispiel 1 beschrieben wurden.

### Beispiel 5

Das Beispiel 1 wurde wiederholt mit der Ausnahme, daß der in Beispiel 1 hergestellten Emulsion noch 1 µg Selen in Form von Na₂SeO₃ x 5H₂O zugesetzt wurden. Bei der i.v.-Verabreichung der so hergestellten Emulsion bei Hauterkrankungen wurden die gleichen Ergebnisse erhalten, wie sie vorstehend für Beispiel 1 geschrieben wurden.

**TABELLE 1**

| Nr. | Pat. | Alter | Geschlecht | Gewicht [kg] | Diagnose | Ausdehnung [%] | Krankheitsdauer |
|---|---|---|---|---|---|---|---|
| A1 | HG | 27 | männlich | 73 | akut-ex. Ps. | 20 | 12 J. |
| A2 | EL | 42 | männlich | 95 | akut-ex. Ps. | 25 | 20 J. |
| A3 | MO | 21 | männlich | 67 | akut-ex. Ps. | 18 | 10 J. |
| A4 | ZH | 47 | männlich | 118 | akut-ex. Ps. | 30 | 20 J. |
| A5 | TF | 30 | männlich | 72 | akut-ex. Ps. | 10 | 20 J. |
| A6 | LJ | 65 | männlich | 66 | akut-ex. Ps. | 10 | ¹/₂ J. |
| A7 | HF | 28 | männlich | 71 | akut-ex. Ps. | 12 | 15 J. |
| A8 | HS | 62 | männlich | 95 | akut-ex. Ps. | 90 | 31 J. |
| A9 | HK | 55 | männlich | 88 | akut-ex. Ps. | 35 | 26 J. |
| A10 | GU | 25 | weiblich | 65 | akut-ex. Ps. | 15 | 2 J. |

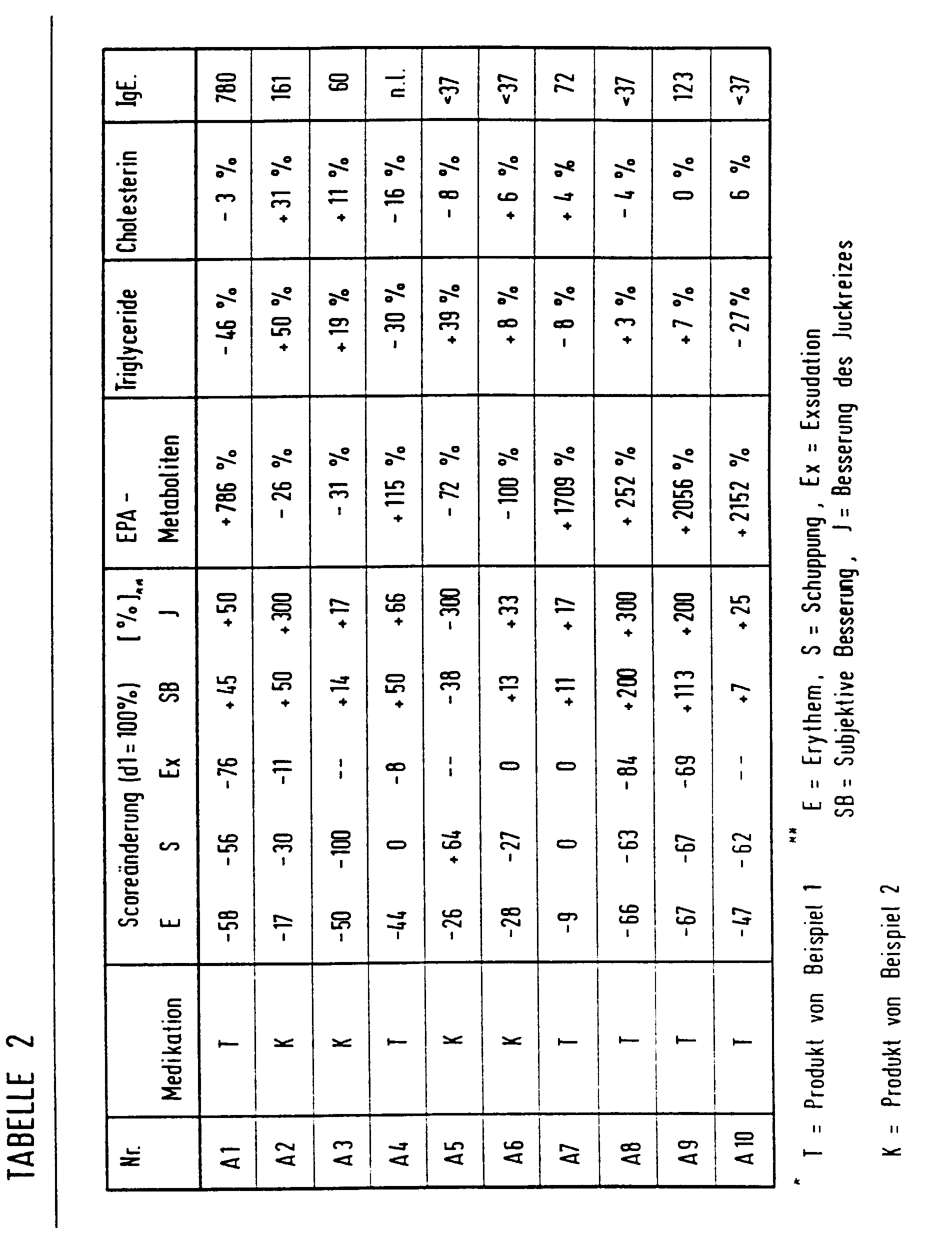

## Patentansprüche (Patentansprüche für folgende(n) Vertragsstaat(en): AT, BE, CH, DE, DK, FR, GB, IE, IT, LI, LU, MC, NL, SE)

1. Verwendung einer Emulsion, enthaltend eine oder mehrere mehrfach ungesättigte, langkettige Omega-3-Fettsäuren und/oder Omega-6-Fettsäuren oder deren pharmazeutisch verträgliche Ester oder Salze sowie übliche Hilfs- und Zusatzstoffe zur Herstellung eines Arzneimittels zur i.v.-Verabreichung zur Behandlung von Hauterkrankungen.

2. Verwendung nach Patentanspruch 1, dadurch gekennzeichnet, daß die Fettsäuren 18 - 22 C-Atome aufweisen.

3. Verwendung nach Patentanspruch 1 oder 2, dadurch gekennzeichnet, daß die Omega-3-Fettsäure α-Linolensäure, Eikosapentaensäure und/oder Docosahexaensäure, vorzugsweise Eikosapentaensäure ist.

4. Verwendung nach Patentanspruch 1 bis 3, dadurch gekennzeichnet, daß die Omega-3-Fettsäuren in Form von Fischölen oder hochgereinigten Fischölkonzentraten oder Leinöl vorliegen.

5. Verwendung nach Patentanspruch 1 bis 4, dadurch gekennzeichnet, daß die Omega-6-Fettsäure Linolsäure, γ-Linolensäure, Di-homo-γ-linolensäure oder Arachidonsäure ist.

6. Verwendung nach Patentanspruch 1 bis 5, dadurch gekennzeichnet, daß die Omega-6-Fettsäuren in Form von Nachtkerzenöl, Borretschöl oder Sojaöl vorliegen.

7. Verwendung nach Anspruch 1 bis 6, dadurch gekennzeichnet, daß die Ester als Ethylester oder Glycerinester, vorzugsweise als Triglyceride vorliegen.

8. Verwendung nach Patentanspruch 1 bis 7, dadurch gekennzeichnet, daß in den Emulsionen die Omega-3-Fettsäuren und/oder Omega-6-Fettsäuren oder deren Ester oder Salze in Mengen von 4 bis 45 Gew.-% vorliegen.

9. Verwendung nach Patentanspruch 8, dadurch gekennzeichnet, daß die Omega-3-Fettsäuren und/oder Omega-6-Fettsäuren oder deren Ester oder Salze in Mengen von 10 bis 30 Gew.-% vorliegen.

10. Verwendung nach Patentanspruch 1 bis 9, dadurch gekennzeichnet, daß als Emulgator Phospholipide pflanzlichen oder tierischen Ursprungs verwendet werden.

11. Verwendung nach Patentanspruch 10, dadurch gekennzeichnet, daß als Emulgator Eilecithin verwendet wird.

12. Verwendung nach Patentanspruch 1 bis 11, dadurch gekennzeichnet, daß der Emulgator in einer Menge von 5 bis 15 Gew.-% (bezogen auf den Fettgehalt) verwendet wird.

13. Verwendung nach Patentanspruch 1 bis 12, dadurch gekennzeichnet, daß die Emulsion in Kombination mit Retinoiden, Cignolin, Photo- bzw. Balneophototherapie, Corticosteroiden (intern/extern), nichtsteroidalen Antiphlogistika und/oder Antihistaminika verwendet wird.

## Patentansprüche (Patentansprüche für folgende(n) Vertragsstaat(en): ES, GR)

1. Verfahren zur Herstellung eines Arzneimittels zur i.v.-Verabreichung zur Behandlung von Hauterkrankungen, dadurch gekennzeichnet, daß man eine Emulsion, enthaltend eine oder mehrere mehrfach ungesättigte, langkettige Omega-3-Fettsäuren und/oder Omega-6-Fettsauren oder deren pharmazeutisch verträgliche Ester oder Salze sowie übliche Hilfs- und Zusatzstoffe verwendet.

2. Verfahren nach Patentanspruch 1, dadurch gekennzeichnet, daß die Fettsäuren 18-22 C-Atome aufweisen.

3. Verfahren nach Patentanspruch 1 oder 2, dadurch gekennzeichnet, daß die Omega-3-Fettsäure α-Linolensäure, Eikosapentaensäure und/oder Docosahexaensäure, vorzugsweise Eikosapentaensäure ist.

4. Verfahren nach Patentanspruch 1 bis 3, dadurch gekennzeichnet, daß die Omega-3-Fettsäuren in Form von Fischölen oder hochgereinigten Fischölkonzentraten oder Leinöl vorliegen.

5. Verfahren nach Patentanspruch 1 bis 4, dadurch gekennzeichnet, daß die Omega-6-Fettsäure Linolsäure, γ-Linolensäure, Di-homo-γ-linolensäure oder Arachidonsäure ist.

6. Verfahren nach Patentanspruch 1 bis 5, dadurch gekennzeichnet, daß die Omega-6-Fettsäuren in Form von Nachtkerzenöl, Borretschöl oder Sojaöl vorliegen.

7. Verfahren nach Anspruch 1 bis 6, dadurch gekennzeichnet, daß die Ester als Ethylester oder Glycerinester, vorzugsweise als Triglyceride vorliegen.

8. Verfahren nach Patentanspruch 1 bis 7, dadurch gekennzeichnet, daß in den Emulsionen die Omega-3-Fettsäuren und/oder Omega-6-Fettsäuren oder deren Ester oder Salze in Mengen von 4 bis 45 Gew.-% vorliegen.

9. Verfahren nach Patentanspruch 8, dadurch gekennzeichnet, daß die Omega-3-Fettsäuren und/oder Omega-6-Fettsäuren oder deren Ester oder Salze in Mengen von 10 bis 30 Gew.-% vorliegen.

10. Verfahren nach Patentanspruch 1 bis 9, dadurch gekennzeichnet, daß als Emulgator Phospholipide pflanzlichen oder tierischen Ursprungs verwendet werden.

11. Verfahren nach Patentanspruch 10, dadurch gekennzeichnet, daß als Emulgator Eilecithin verwendet wird.

12. Verfahren nach Patentanspruch 1 bis 11, dadurch gekennzeichnet, daß der Emulgator in einer Menge von 5 bis 15 Gew.-% (bezogen auf den Fettgehalt) verwendet wird.

13. Verfahren nach Patentanspruch 1 bis 12, dadurch gekennzeichnet, daß die Emulsion in Kombination mit Retinoiden, Cignolin, Photo- bzw. Balneophototherapie, Corticosteroiden (intern/extern), nichtsteroidalen Antiphlogistika und/oder Antihistaminika verwendet wird.

## Claims (Claims for the following Contracting State(s): AT, BE, CH, DE, DK, FR, GB, IE, IT, LI, LU, MC, NL, SE)

1. Use of an emulsion containing one or more polyunsaturated, long-chain Omega-3 fatty acids or their pharmaceutically compatible esters or salts, together with the usual accessories and additives for the manufacture of a drug for i.v. administration in the treatment of skin diseases.

2. Use as per claim 1, characterized by the fact that the fatty acids have 18 - 22 C-atoms.

3. Use as per claim 1 or 2, characterized by the fact that the Omega-3 fatty acid is α-linolenic acid EPA and/or DHA, preferably EPA.

4. Use as per claims 1 to 3, characterized by the fact that the Omega-3 fatty acids are present in the form of fish oils or highly refined fish oil concentrates or linseed oil.

5. Use as per claims 1 to 4, characterized by the fact that the Omega-6 fatty acid is linoleic acid, γ-linolenic acid, dihomo-γ-linolenic acid or arachidonic acid.

6. Use as per claims 1 to 5, characterized by the fact that the Omega-6 fatty acids are present in the form of evening primrose oil, dill oil or soya oil.

7. Use as per claims 1 to 6, characterized by the fact that the esters are present as ethyl ester or glycerine ester, preferably as triglyceride.

8. Use as per claims 1 to 7, characterized by the fact that in the emulsions the Omega-3 fatty acids and/or Omega-6 fatty acids or their esters or salts are present in quantities from 4 to 45% by weight.

9. Use as per claim 8, characterized by the fact that the Omega-3 fatty acids and/or Omega-6 fatty acids are present in quantities ranging from 10 to 30% by weight.

10. Use as per claims 1 to 9, characterized by the fact that phospholipids of vegetable or animal origin are present as the emulsifying agent.

11. Use as per claim 10, characterized by the fact that egg lecithin is used as the emulsifying agent.

12. Use as per claims 1 to 11, characterized by the fact that the emulsifying agent is used in a quantity ranging from 5 to 15% by weight (in relation to the fat content).

13. Use as per claims 1 to 12, characterized by the fact that the emulsion is used in combination with retinoids, Cignolin, phototherapy or balneo-phototherapy, corticosteroids (internally/externally), non-steroidal antiphlogisitics and/or antihistamines.

## Claims (Claims for the following Contracting State(s): ES, GR)

1. Process for the manufacture of a drug for i.v. administration in the treatment of skin diseases, characterized by the fact that an emulsion is used, containing one or more polyunsaturated, long-chain Omega-3 fatty acids and/or Omega-6 fatty acids or their pharmaceutically compatible esters or salts, together with the usual accessories and additives.

2. Process as per claim 1, characterized by the fact that the fatty acids display 18 - 22 C-atoms.

3. Process as per claim 1 or 2, characterized by the fact that the Omega-3 fatty acid is α-linolenic acid, EPA and/or DHA, preferably EPA.

4. Process as per claims 1 to 3, characterized by the fact that the Omega-3 fatty acids are present in the form of fish oils or highly purified fish oil concentrates or linseed oil.

5. Process as per claims 1 to 4, characterized by the fact that the Omega-6 fatty acid is linoleic acid, γ-linolenic acid, Di-homo-γ-linolenic acid or arachidonic acid.

6. Process as per claims 1 to 5, characterized by the fact that the Omega-6 fatty acids are present in the form of evening primrose oil, dill oil or soya oil.

7. Process as per claims 1 to 6, characterized by the fact that the esters are present as ethyl esters or glycerine esters, preferably as triglyceride.

8. Process as per claims 1 to 7, characterized by the fact that in the emulsions the Omega-3 fatty acids and/or the Omega-6 fatty acids or their esters or salts are present in quantities ranging from 4 to 45% by weight.

9. Process as per claim 8, characterized by the fact that the Omega-3 fatty acids and/or the Omega-6 fatty acids or their esters or salts are present in quantities ranging from 10 to 30% by weight.

10. Process as per claims 1 to 9, characterized by the fact that phospholipids of vegetable or animal origin are used as the emulsifying agent.

11. Process as per claim 10, characterized by the fact that egg lecithin is used as the emulsifying agent.

12. Process as per claims 1 to 11, characterized by the fact that the emulsifying agent is used in a quantity ranging from 5 to 15% by weight (in relation to the fat content).

13. Process as per claims 1 to 12, characterized by the fact that the emulsion is used in combination with retinoids, Cignolin, phototherapy and/or balneo-phototherapy, corticosteroids (internal/external), non-steroidal antiphlogistics and/or antihistamines.

## Revendications (Revendications pour l'(les) Etat(s) contractant(s) suivant(s): AT, BE, CH, DE, DK, FR, GB, IE, IT, LI, LU, MC, NL, SE)

1. Utilisation d'une émulsion contenant un ou plusieurs acides gras oméga-3 et/ou acides gras oméga-6 à longues chaînes plusieurs fois insaturés ou encore leurs esters ou leurs sels pharmaceutiquement acceptables, ainsi que des adjuvants et des additifs habituels pour la préparation d'un médicament destiné à l'administration par voie intraveineuse pour le traitement de maladies de la peau.

2. Utilisation selon la revendication 1, caractérisée en ce que les acides gras présentent de 18 à 22 atomes de carbone.

3. Utilisation selon la revendication 1 ou 2, caractérisée en ce que l'acide gras oméga-3 est l'acide α-linolénique, l'acide éicosapentaénoïque et/ou l'acide docosahexaénoïque, de préférence l'acide éicosapentaénoïque.

4. Utilisation selon les revendications 1 à 3, caractérisée en ce que les acides gras oméga-3 sont présents sous la forme d'huiles de poissons ou de concentrats d'huiles de poissons à pureté élevée ou encore d'huile de lin.

5. Utilisation selon les revendications 1 à 4, caractérisée en ce que l'acide gras oméga-6 est l'acide linoléique, l'acide γ-linolénique, l'acide di-homo-γ-linolénique ou l'acide arachidonique.

6. Utilisation selon les revendications 1 à 5, caractérisée en ce que les acides oméga-6 sont présents sous la forme d'huile d'onagre, d'huile de bourrache ou d'huile de soja.

7. Utilisation selon les revendications 1 à 6, caractérisée en ce que les esters sont présents sous forme d'esters éthyliques ou d'esters glycériques, de préférence sous forme de triglycérides.

8. Utilisation selon les revendications 1 à 7, caractérisée en ce que les acides gras oméga-3 et/ou les acides gras oméga-6 ou encore leurs esters ou leurs sels sont présents dans les émulsions dans des quantités de 4 à 45% en poids.

9. Utilisation selon la revendication 8, caractérisée en ce que les acides gras oméga-3 et/ou les acides gras oméga-6 ou encore leurs esters ou leurs sels sont présents dans des quantités de 10 à 30% en poids.

10. Utilisation selon les revendications 1 à 9, caractérisée en ce qu'on utilise comme émulsifiant des phospholipides d'origine végétale ou animale.

11. Utilisation selon la revendication 10, caractérisée en ce qu'on utilise comme émulsifiant la lécithine de l'oeuf.

12. Utilisation selon les revendications 1 à 11, caractérisée en ce qu'on utilise l'émulsifiant en une quantité de 5 à 15% en poids (rapportés à la teneur en matières grasses).

13. Utilisation selon les revendications 1 à 12, caractérisée en ce qu'on utilise l'émulsion en combinaison avec des rétinoïdes, avec la cignoline, avec la photo-, respectivement la balnéophotothérapie, avec des corticostéroïdes (par voie interne/externe), avec des antiphlogistiques non stéroïdiens et/ou des antihistaminiques.

## Revendications (Revendications pour l'(les) Etat(s) contractant(s) suivant(s): ES, GR)

1. Procédé pour la préparation d'un médicament destiné à l'administration par voie intraveineuse pour le traitement de maladies de la peau, caractérisé en ce qu'on utilise une émulsion contenant un ou plusieurs acides gras oméga-3 et/ou acides gras oméga-6 à longues chaînes plusieurs fois insaturés ou encore leurs esters ou leurs sels pharmaceutiquement acceptables, ainsi que des adjuvants et des additifs habituels.

2. Procédé selon la revendication 1, caractérisé en ce que les acides gras présentent de 18 à 22 atomes de carbone.

3. Procédé selon la revendication 1 ou 2, caractérisé en ce que l'acide gras oméga-3 est l'acide α-linolénique, l'acide éicosapentaénoïque et/ou l'acide docosahexaénoïque, de préférence l'acide éicosapentaénoïque.

4. Procédé selon les revendications 1 à 3, caractérisé en ce que les acides gras oméga-3 sont présents sous la forme d'huile de poisson ou de concentrats d'huile de poisson à purification élevée ou encore d'huile de lin.

5. Procédé selon les revendications 1 à 4, caractérisé en ce que l'acide gras oméga-6 est l'acide linoléique, l'acide γ-linolénique, l'acide di-homo-γ-linolénique ou l'acide arachidonique.

6. Procédé selon les revendications 1 à 5, caractérisé en ce que les acides oméga-6 sont présents sous la forme d'huile d'onagre, d'huile de bourrache ou d'huile de soja.

7. Procédé selon les revendications 1 à 6, caractérisé en ce que les esters sont présents sous forme d'esters éthyliques ou d'esters glycériques, de préférence sous forme de triglycérides.

8. Procédé selon les revendications 1 à 7, caractérisé en ce que les acides gras oméga-3 et/ou les acides gras oméga-6 ou encore leurs esters ou leurs sels sont présents dans les émulsions dans des quantités de 4 à 45% en poids.

9. Procédé selon la revendication 8, caractérisé en ce que les acides gras oméga-3 et/ou les acides gras oméga-6 ou encore leurs esters ou leurs sels sont présents dans des quantités de 10 à 30% en poids.

10. Procédé selon les revendications 1 à 9, caractérisé en ce qu'on utilise comme émulsifiant des phospholipides d'origine végétale ou animale.

11. Procédé selon la revendication 10, caractérisé en ce qu'on utilise comme émulsifiant la lécithine de l'oeuf.

12. Procédé selon les revendications 1 à 11, caractérisé en ce qu'on utilise l'émulsifiant en une quantité de 5 à 15% en poids (rapportés à la teneur en matières grasses).

13. Procédé selon les revendications 1 à 12, caractérisé en ce qu'on utilise l'émulsion en combinaison avec des rétinoïdes, avec la cignoline, avec la photo-, respectivement la balnéophotothérapie, avec des corticostéroïdes (par voie interne/externe), avec des antiphlogistiques non stéroïdiens et/ou des antihistaminiques.
